**Europäisches Patentamt**

**European Patent Office**

**Office européen des brevets**

(19)

(11) Veröffentlichungsnummer: **0 214 392**
**B1**

(12) **EUROPÄISCHE PATENTSCHRIFT**

(45) Veröffentlichungstag der Patentschrift: **23.05.90**

(21) Anmeldenummer: **86108875.5**

(22) Anmeldetag: **30.06.86**

(51) Int. Cl.⁵: **A 61 M 1/34,** A 61 M 1/36,
A 61 L 27/00, A 61 L 29/00,
A 61 L 31/00

(54) **Medizinisches Gerät, insbesondere Filter, Kanüle, Katheter oder Implantat.**

(30) Priorität: **02.07.85 DE 3523615**
**02.07.85 DE 3523616**

(43) Veröffentlichungstag der Anmeldung:
**18.03.87 Patentblatt 87/12**

(45) Bekanntmachung des Hinweises auf die
Patenterteilung:
**23.05.90 Patentblatt 90/21**

(84) Benannte Vertragsstaaten:
**AT BE CH DE FR GB IT LI LU NL SE**

(56) Entgegenhaltungen:
**EP-A-0 082 345**
**EP-A-0 108 658**
**EP-A-0 138 297**
**WO-A-84/01108**
**AT-E- 10 583**
**DD-A- 220 500**
**DE-A-2 539 657**
**DE-A-2 738 183**
**DE-A-2 812 174**
**DE-A-3 321 629**

(73) Patentinhaber: **CYTOMED Medizintechnik
GmbH
Fabrikstrasse 20
D-8750 Aschaffenburg (DE)**

(72) Der Erfinder haben auf ihre Nennung verzichtet

(74) Vertreter: **Patentanwälte Dr. Solf & Zapf
Zeppelinstrasse 53
D-8000 München 80 (DE)**

(56) References cited:
**US-A-3 848 580**
**US-A-4 031 201**
**US-A-4 253 844**

**Chromatography, Electrophoresis,
Immunochemistry, Bro-Rad; katalog k 1985**

Courier Press, Leamington Spa, England.

## Beschreibung

Die Erfindung betrifft ein medizinisches Gerät, insbesondere Filter, Kanüle, Katheter oder Implantat, aus Kunststoff oder Kunststoffbeschichtetem Metall oder Glas, mit dessen Hilfe innerhalb und außerhalb des meschlichen Körpers krankheitserregende Keime, insbesondere Viren, Bakterien und Pilze, sowie pathogene Stoffwechselprodukte, Toxine, Fettkörper und Antikörper fixiert und damit unschädlich gemacht werden können, bei dem die Kunststoffoberfläche mit fest daran gebundenen oder kovalent damit verbundenen Antikörpern ausgestattet ist. Das erfindungsgemäße medizinische Gerät kann in den menschlichen Körper eingeführt werden, ohne daß die Gefahr einer Infektion besteht.

Aus der DE—OS—32 28 849 ist ein in den Körper einzuführendes medizinisches Gerät mit einem Überzug bekannt, bei dem der Überzug mikrobentötende Metallionen abgibt. Die Metallionen bestehen aus Gold-, Silber- und Kupferionen.

Es ist auch bereits bekannt, bei in den menschlichen Körper einzuführenden Geräten, wie z.B. Sonden, deren Oberfläche mit Jodverbindungen zu behandeln, um einer Infektionsgefahr vorzubeugen.

Aus EP—A—82 345, von der die Erfindung ausgeht, ist ein medizinisches Gerät aus Kunststoff bekannt, dessen Oberfläche mit Gamma-Immunoglobulinen besetzt ist, das dazu dient, gesundheitsschädliche Komponenten aus dem perfundierten Blut zu entfernen mittels der gegen die Antigene der krankheitserregenden Keime wirksamen Gamma-Immunoglobuline.

Aus EP—A—138 297 ist es bekannt, daß Kunststoffteilchen mit daran gebundenen monoklonalen Immunoglobulinen zum Fixieren von Antigenen, gegen die die Immunoglobuline wirksam sind, verwendet werden können. Die darin beschriebene Suspension von diagnostischen Kunststoffteilchen aus einem Carboxylat-Polymeren und daran fixierten Antikörper-Molekülen dient in erster Linie der Durchführung von Agglutinationstests.

Alle bekannten medizinischen Geräte des vorstehend beschriebenen Typs haben sich in der Praxis jedoch als unzulänglich erwiesen, da die auf ihre Oberfläche aufgebrachten Mittel, wie z.B. Edelmetallionen, Jodverbindungen, Antibiotika oder Gamma-Globuline, von dem beschichteten Material abgegeben werden, so daß damit die Infektionsgefahr nur kurzfristig vermindert wird. Hinzu kommt, daß die vorhandenen Keime mit den abgegebenen Substanzen in Wechselwirkung treten können und daß es dadurch u.a. zu einer Resistenzbildung kommen kann. Über die Resistenzbildung hinaus können die dabei auftretenden Stoffwechselprodukte auf den Organismus auch eine negative Wirkung haben.

Neben dem Infektionsrisiko wirkt sich bei den in den Körper einzuführenden medizinischen Geräten, insbesondere Kathetern, die Bildung einer Fibrinschicht auf der Oberfläche bei intravasalen Kathetern negativ aus. Um diesem Mangel abzuhelfen, wurde bereits vorgeschlagen, intravasale Katheter mit Heparin zu beschichten, um die unerwünschten Vibrinablagerungen zu vermeiden. Auch dieser Vorschlag hat sich aber in der Praxis als nicht erfolgreich erwiesen.

Die zunehmende Entwicklung von Mehrfachresistenzen und der damit verbundene hohe finanzielle Aufwand erfordern ein neues Schutzsystem. Es sind daher medizinische Geräte, insbesondere Filter, Kanülen, Katheter und Implantate, erwünscht, die auch langfristig im Körper verbleiben können, ohne daß von ihnen eine Infektionsgefahr ausgeht.

In der Medizin wird ferner seit langem versucht, Behandlungsschemata für hochtoxische, virale oder bakterielle Infektionskrankheiten, wie Aids, Hepatitis A, Hepatitis B, Hepatitis Non-A, Hepatitis Non-B, Tetanus und genetisch bedingte Stoffwechselerkrankungen, wie Phenylketonurie oder andere Vergiftungen, z.B. mit Digitalis-Glykosiden oder Barbituraten, zu entwickeln. Die Erfolge sind jedoch äußerst gering. Als bekanntes Mittel gegen virale Erkrankungen wird Interferon eingesetzt. Die Effektivität der Interferon-Behandlung ist jedoch nur gering, da die Spektren der verschiedenen Interferone und deren Organspezifitäten äußerst mannigfaltig sind.

Auch bei bakteriell bedingten Krankheitsbildern, wie Tetanus, genetisch bedingten Stoffwechselerkrankungen, wie Phenylketonurie oder Vergiftungen, z.B. mit Digitalis-Glykosiden oder Barbituraten, sind die bisherigen Therapieerfolge trotz erheblichem materiellem und personellem Aufwand gering.

Es bestand daher seit langem der Wunsch nach einer einfachen Methode, mit der die krankheitserregenden Viren, Bakterien, Pilze, pathogenen Stoffwechselprodukte, Fettkörper (insbesondere Lipide) und Arzneimittel aus dem Blut entfernt bzw. herausgefiltert werden können. Die Behandlungsmethode sollte jedoch so gestaltet sein, daß die krankheitserregenden Stoffe oder sonstigen Stoffe, falls sie herausgefiltert werden können, auch nicht als Antigen-Antikörper-Komplex im Blut verbleiben.

Aufgabe der Erfindung war es daher, ein medizinisches Gerät, insbesondere Filter, Kanüle, Katheter oder Implantat, aus Kunststoff oder kunststoffbeschichtetem Metall oder Glas zu entwickeln, mit dessen Hilfe es möglich ist, innerhalb und außerhalb des menschlichen Körpers krankheitserregende Keime, insbesondere Viren, Bakterien und Pilze, sowie pathogene Stoffwechselprodukte, Toxine, Fettkörper (speziell Lipide) und Arzneimittel im Blut, insbesondere menschlichem Blut, auf einfache Weise so zu fixieren, daß dadurch bedingte Infektionen zuverlässig vermieden werden können bzw. diese unerwünschten Stoffe aus dem Blut zuverlässig und gefahrlos entfernt werden können.

Es wurde nun gefunden, daß diese Aufgabe gelöst werden kann durch ein medizinisches Gerät, insbesondere Filter, Kanüle, Katheter oder Implantat, aus Kunststoff oder kunststoffbeschichtetem Metall oder Glas zur Fixierung krankheitserregender Keime, insbesondere Viren, Bakterien und Pilze, sowie pathogener Stoffwechselprodukte, Toxine, Fettkörper und Arzneimittel, bei dem die Kunststoffoberfläche mit fest daran gebundenen oder kovalent damit verbundenen Antikörpern ausgestattet ist, das dadurch gekennzeichnet ist, daß es sich bei den Antikörpern handelt um die F(ab)$_2$-Teile, vorzugsweise die durch Affinitätschromatographie nachgereinigten F(ab)$_2$-Teile, von homologen oder monoklonalen Immunglobulinen der Klassen G1, G2, G3 und/oder G4 und/oder IGM, IGE, IGD und/oder IGA, die selektiv wirksam sind gegen die spezifischen Antigene oder antigenen Determinanten der jeweiligen krankheitserregenden Keime, insbesondere Viren, Bakterien und Pilze, sowie der pathogenen Stoffwechselprodukte, Toxine, Fettkörper (z.B. Lipide) und Arzneimittel.

Als Kunststoffmaterial werden in der Medizintechnik übliche Kunststoffe verwendet, vorzugsweise Polyolefine, Polyester, Polyäther, Polyamide, Polyimide, Polyurethane, Polyvinylchloride, Polysulfone, Polystyrole, Polyacrylate, Polymethacrylate, Polypropylene, Polyvinylpyrrolidone, Fluorpolymere, Kunststoffe auf Basis von Acrolein, Derivate dieser Verbindungen, Mischungen und Copolymere derselben sowie Silikonkautschuke.

Besonders geeignet sind Kunststoffe auf Polystyrol- und Polyurethan-Basis. Des wieteren eignet sich dazu mit beispielsweise Acrolein vorbehandeltes Glas, wie Controlporeglas.

Die Oberfläche des erfindungsgemäß verwendeten Kunststoffs wird vorzugsweise mit γ-Strahlen oder Ozon vorbehandelt, da dadurch die Haftfestigkeit der aufgebrachten Antikörper erhöht wird. Es ist auch eine Vorbehandlung der Kunststoffoder Glasoberfläche durch Ionenätzung oder mittels Acrolein oder einer Säure möglich.

Besonders vorteilhaft ist das Aufbringen eines Haftvermittlers oder Spacers auf die Kunststoffoberfläche zur Verbesserung der Haftung der Antikörper an der Kunststoffoberfläche, insbesondere eines solchen aus der Gruppe Bromcyan, Thiophosgen und Thionylchlorid, die auch zur Aktivierung der Kunststoffoberfläche verwendet werden können und die auch eine kovalente Bindung zwischen Substrat und Antikörper erzeugen können.

Spacers sind molekulare Zwischenstücke, um die Abstände zwischen der Oberfläche des Trägermaterials und den Antikörpern zu vergrößern. Die Bezeichnung Spacers ist dem Fachmann geläufig. Die kovalente Bindung wird beispielsweise mittels Bromcyan oder Thiophosgen erzielt.

Die Erfindung wird nachstehend anhand zweier bevorzugter Ausführungsformen, nämlich eines Filters zur Abtrennung von krankheitserregenden Keimen, insbesondere Viren, Bakterien und Pilzen, sowie pathogenen Stoffwechselprodukten, Fettkörpern (z.B. Lipiden) und Arzneimitteln aus Blut, einerseits und Kathetern, Kanülen oder Implantaten, die langfristig im Körper verbleiben können, ohne daß Infektionsgefahr besteht, andererseits, näher erläutert.

Es wurde nunmehr ein Filter der eingangs angegebenen Art entwickelt, das dadurch gekennzeichnet ist, daß es aus einem von Blut durchströmbaren, physiologisch umbedenklichen Kunststoff besteht, wobei die Kunststoffoberfläche des Filters besetzt ist mit den F(ab)$_2$-Teilen, vorzugsweise den durch Affinitätschromatographie nachgereinigten F(ab)$_2$-Teilen, von homologen oder monoklonalen Immunglobulinen der Klasse G1, G2, G3 und/oder G4, und/oder IGM, IGE, IGD und/oder IGA, die selektiv wirksam sind gegen die spezifischen Antigene oder antigenen Determinanten der jeweiligen Viren, Bakterien, Pilze, pathogenen Stoffwechselprodukte, Toxine, Fettkörper und Arzneimittel. Diese Antikörper sind an die Oberfläche des Filters durch Adsorption oder Haftvermittler oder kovalente Bindung fest gebunden.

Die Kunststoffoberfläche des Filters ist vorzugsweise eine mit Gamma-Strahlen oder Ozon vorbehandelte Oberfläche, da dadurch die Haftfestigkeit der durch ein Tränkungsverfahren aufgebrachten Antikörper erhöht wird. Die Haftung der F(ab)$_2$-Teile der Immunglobuline der Klasse G1, G2, G3 und/oder G4 und/oder IGM, IGE, IGD und/oder IGA auf der Kunststoffoberfläche des Filters kann dadurch noch verbessert werden, daß nach der Oberflächenbehandlung mit Gamma-Strahlung oder Ozon ein Haftvermittler oder Acrolein auf die Oberfläche aufgebracht wird, der die Haftung der F(ab)$_2$-Teile der Immunglobuline auf der Kunststoffoberfläche verbessert, speziell durch Ausbildung einer kovalenten Bindung.

Das Filter kann aus einem Kompaktfilter, Membranfilter, Füllkörperfilter, Molekularsiebfilter oder Kapillarfilter bestehen. Besonders geeignet ist auch ein Gehäuse, das mit kugelförmigen, ringförmigen, sattelförmigen, spiralförmigen, sternförmigen, zylinderförmigen oder netzförmigen Kunststoffteilchen gefüllt ist. Auch die bekannten Kapillarhämofilter sind für den erfindungsgemäßen Zweck einsetzbar.

Die Abtrennung der krankheitserregenden und sonstigen Stoffe aus dem Blut erfolgt in der Form, daß das Blut aus dem Körper über eine Blutpumpe in das Filter gedrückt wird, wobei die Filtrationsgeschwindigkeit etwa 6 bis 30 ml Blut pro Minute beträgt. Das gesamte Blutvolumen des Patienten, der z.B. an Aids, Hepatitis A, Hepatitis B, Hepatitis Non-A, Hepatitis Non-B, einer viralen Encephalitis oder an einer anderen hochpathogenen therapieresistenten, bakteriellen oder mykotischen Infektion oder einer genetisch bedingten, virusinduzierten oder genetischen Stoffwechselerkrankung, wie z.B. Phenylketonurie oder an einer Intoxikation erkrankt ist, wird ähnlich wie bei der Hämodialyse über das erfindungsgemäße Filter geleitet.

Dabei erfolgt durch die auf die Kunststoffoberfläche des erfindungsgemäßen Filters aufgebrachten F(ab)$_2$-Teile der homologen oder monoklonalen Immunglobuline die jeweils spezifische Antigen-Antikörper-Komplexbildung, d.h. daß die jeweiligen Viren, Bakterien, pathogenen Stoffwechselprodukte, Toxine, Fettkörper und Arzneimittel durch die Bindung an die Antikörper aus dem durch das Filter strömenden Blut herausgefiltert werden.

Die obengenannten pathogenen oder anderen pathogenen Substanzen oder Organismen werden auf diese Weise auf dem Filter immobilisiert. Der Antigen-Antikörper-Komplex wird somit nicht von dem Blutstrom mitgerissen.

Die Reaktionsfläche, d.h. die Größe der mit Antikörper beschichteten Filterfläche, wird auf der Grundlage der jeweiligen Filter ermittelt. Das auf diese Weise gereinigte Blut wird im Anschluß daran retransfundiert.

Die Blutwäsche erfolgt bei etwa 37°C und wird bei krankheitserregenden Bakterien und Pilzen zwei- bis dreimal und bei der Abtrennung von krankheitserregenden Viren fünf- bis sechsmal wiederholt, jedoch solange, bis die Erreger vollständig aus dem Blut entfernt sind. Bei der Beseitigung der Viren ist die Wiederholung der Blutwäsche nach der Lyse der Zellen vorzunehmen, d.h. der jeweilige Zyklus der Lyse, die sich durch einen entsprechenden Temperaturanstieg zeigt, wird als Maß für die nächste Blutwäsche genommen. Die wiederholte Anwendung der Blutwäsche bei Viren, die an DNS gebunden sind, ist deshalb notwendig, da eine Reaktion dieser Viren mit den Antikörpern auf der Oberfläche des Filters erst nach der entsprechenden Lyse der Zellen erfolgen kann.

Bei hochpathogenen therapieresistenten Keimen und bei Intoxikationen, die nicht an DNS gebunden sind, ist im allgemeinen eine ein- bis zweimalige Durchleitung des infizierten Blutes durch das erfindungsgemäße Filter ausreichend. Bei genetisch bedingten Stoffwechselerkrankungen ist die Blutwäsche je nach Höhe der angefallenen Konzentration der pathogenen Stoffwechselprodukte zu wiederholen.

Das Aufbringen der $F(ab)_2$-Teile der Immunglobuline G1, G2, G3 und/oder G4 und/oder IGM, IGE, IGD und/oder IGA auf die Kunststoffoberfläche des Filters erfolgt durch Eintauchen in die entsprechende Immunglobulinlösung. Die Tränkungszeiten betragen etwa 1 bis 10 Stunden. Danach wird das mit den Antikörpern besetzte Filter mit einer Phosphatpufferlösung gewaschen, um die überschüssigen Antikörper abzuwaschen. Dann wird das Filter getrocknet.

Alternativ zu dem obengenannten Tränkungsverfahren können die genannten Antikörper über aktivierte Kunststoffe mittels Bromcyan, Thiophosgen oder Thionylchlorid nach einer Gamma-Bestrahlung durch Haftvermittler oder ein ähnliches, kovalente Bindungen erzeugendes Verfahren, aufgebracht werden.

Die bisher durchgeführte Blutwäsche geht davon aus, daß komplette Antikörper mit den jeweiligen Antigenen der zu entfernenden Substanzen zu einem Antigen-Antikörper-Komplex reagieren. Eine Optimierung dieses Verfahrens wird gerade bei der Behandlung von Infektionskrankheiten wie z.B. Aids dadurch erreicht, daß die Antikörper mit Pepsin vorbehandelt werden, wodurch eine Aufspaltung in $F(ab)_2$-und Fc-Teil erfolgt. Anschließend wird der Fc-Teil mittels Chromatographie abgetrennt. Hierzu wird Sepharose CL6B verwendet. Das aktivierte Gel wird dabei über einen Zeitraum von 40 bis 54 Stunden bei 2 bis 10°C reagieren gelassen, wodurch die Abtrennung des Fc-Teils quantitativ erfolgt. Die erhaltenen $F(ab)_2$-Teile werden nun mit den Kunststoffoberflächen reagieren gelassen. Als Trägermaterial werden neben polaren Kunststoffen auch Glaskugeln verwendet. Die Bedeutung der Blutwäsche mittels der $F(ab)_2$-Teile der Immunglobuline ist in folgendem begründet:

1. Bei der Reaktion von Vollblut mit dem nicht aufgetrennten Immunglobulin erfolgt neben der spezifischen Antigen-Bindung am $F(ab)_2$-Teil eine Anzahl unspezifischer Bindungen am Fc-Teile. Eine wichtige Gruppe stellen dabei u.a. die Komplementfaktoren dar. Für die praktische Anwendung bedeutet dies, daß dem ohnehin geschwächten Patienten wichtige Substanzen für die körpereigene Infektionsabwehr entzogen werden. Die Phagozytose der jeweiligen Keime wird erheblich eingeschränkt.

Die Problematik eventueller Thrombenbildung bei der Durchführung der beschriebenen Blutwäsche wird dadurch ausgeschaltet, daß 5—25% der beschichteten Reaktionsfläche nicht mit dem $F(ab)_2$-Teil, sondern mit Heparin besetzt werden. Im Gegensatz zu den $F(ab)_2$-Molekülen, die kovalent gebunden sind, wird das Heparin durch Adsorption aufgebracht. Die Sterilisation der Filtrationseinheit zur Blutwäsche ist auf Grund des Proteincharakters der aktiven Antikörper mit Äthylenoxid bzw. Gamma-Bestrahlung äußest schonend durchzuführen, wobei eine Gewährleistung für eine nicht auftretende Inaktivierung der aktiven Antikörper nicht gegeben werden kann.

Zur sicheren Vermeidung der Inaktivierung bietet sich folgendes neues Sterilisationsverfahren an:

Eine Mischung heterologer Immunglobuline wird in eine physiologische Lösung (z.B. Ringerlösung) gebracht. Mit dieser Lösung wird die Filtereinheit versetzt und über einen Zeitraum von 10—30 Minuten belassen. Auf Grund der heterologen Immunglobuline kommt es mit potentiell vorhandenen Keimen oder Pyrogenen zu einer Antigen-Antikörper-Reaktion mit unterschiedlicher Avidität. Die Größe des Antigen-Antikörper-Komplexes erfordert ein Ausspülen der so gebundenen Keime bzw. Pyrogene durch anschließende 3-malige Spülung mit einer physiologischen Lösung ohne Antikörper.

Medizinische Kanülen, Katheter oder Implantate gemäß der Erfindung sind dadurch gekennzeichnet, daß an der Kunststoffoberfläche die o.g. Antikörper adsorbiert sind.

Die Basisüberlegung betrifft dabei die Tatsache, daß alle Keime, ob Bakterien oder Viren, über eine Anzahl von Antigendeterminanten verfügen. Im Organismus bewirken diese die Produktion von spezifischen Antikörpern, die den für den Gesundungsprozeß nötigen Antigen-Antikörper-Komplex bilden. Allein durch die Bildung des Antigen-Antikörper-Komplexes auf der Kunststoffoberfläche erfolgt eine Immobilisierung der Keime. Es wird somit verhindert, daß die Keime in den Körper weiter vordringen. Bei einer entsprechenden Beschichtung von Implantaten, Kathetern, Kanülen und Sondenmaterialien sowie künstlichen Organen mit Antikörpern kann die Antigen-Antikörper-Komplexbildung auf der Oberfläche des Kunststoffes in vivo erreicht werden. Dies bedeutet, daß Keime beim Kontakt mit den Antikörpern aufgrund ihrer antigenen Eigenschaften bebunden werden und in ihrer Mobilisation inhibiert sind. Ein weiteres Eindringen oder Vermehren im Organismus wird somit ausgeschlossen.

Da die Antikörper fest, vorzugsweise kovalent auf der Kunststoffoberfläche gebunden sind und nicht in das Medium abgegeben werden, erfolgt auch keine Verstoffwechselung, da der beschriebene Effekt immobilisierend und nicht bakterizid bzw. bakteriostatisch ist.

Durch allseitige Beschichtung der beschriebenen Katheter, Kanülen und Implantate wird auch eine retrograde Keimwanderung an der Außen- und Innenwand der aufgezählten Artikel inhibiert.

Die Kunststoffoberfläche bzw. das Kunststoffmaterial besteht vorzugsweise aus Polypropylen, Polyurethan, Polyvinylpyrrolidon, Polyacrylat, Polymethacrylat, Polyamid oder den Copolymeren davon.

Die Kunststoffoberfläche wird vor dem Auftragen der Antikörper mittels γ-Strahlen, Ionenätzung, Ozonätzung oder mit einer Lösung von einem pH-Wert von 7,5 bis 10,5 oder mit Acrolein vorbehandelt, um die Haftfestigkeit der Antikörper an der Oberfläche zu verbessern. Die Behandlung wird je nach Kunststoffausgangsmaterial bei einem pH-Wert von 7,5 bis 10,5 durchgeführt, wobei die Reaktionszeit ebenfalls in Abhängigkeit vom Kunststoff 5 bis 10 Minuten beträgt. Die Ausfrauhung der zu beschichtenden Kunststoffoberfläche kann insbesondere auch durch eine Alkylierungsbehandlung mit Methyljodid vorgenommen werden.

Zusätzlich zu der Aufrauhung der Oberfläche kann der Kunststoff auch noch mit einem Haftvermittler, insbesondere Bromcyan, Thiophosgen oder Thionylchlorid, behandelt werden, der ebenfalls die Haftung der Antikörper an der Kunststoffoberfläche bewirkt, insbesondere durch Ausbildung einer kovalenten Bindung.

Eine weitere Möglichkeit zum Aufbringen der Antikörper auf die Kunststoffoberfläche der Kanülen, Katheter, Implantate, Sonden, Gefäßprothesen und Herzschrittmacher erfolgt durch Tränken der durch entsprechende Aufrauhungsbehandlung vorbehandelten Kunststoffoberfläche mit der gesättigten Antikörper lösung. Das Tränken der Kunststoffoberfläche wird bei etwa 20 bis 40°C vorgenommen, und zwar über eine Zeitraum von etwa 1 bis 10 Stunden.

Nach der Bindung mittels Haftvermittler oder der Adsorption der Antikörper auf der Kunststoffoberfläche werden die Trägermaterialien mit einer Phosphatpufferlösung gewaschen, um überschüssige Antikörper abzuspülen. Danach werden die Materialien bei entsprechender Temperatur (20 bis 40°C für 8 bis 24 Stunden) getrocknet und dann steril verpackt.

Als Kunststoffmaterialien werden nur polare Kunststoffe verwendet, die für eine Einbringung in den Körper die notwendigen physikalischen Eigenschaften, wie Elastizität und Bruchstabilität, aufweisen.

**Patentansprüche**

1. Medizinisches Gerät, insbesondere Filter, Kanüle, Katheter oder Implantat, aus Kunststoff oder kunststoffbeschichtetem Metall oder Glas zur Fixierung krankheitserregender Keime, insbesondere von Viren, Bakterien und Pilzen, sowie pathogener Stoffwechselprodukte, Toxine, Fettkörper und Arzneimittel, bei dem die Kunststoffoberfläche mit fest daran gebundenen oder kovalent damit verbundenen Antikörpern ausgestattet ist, dadurch gekennzeichnet, daß es sich bei den Antikörpern handelt um die $F(ab)_2$-Teile, vorzugsweise die durch Affinitätschromatographie nachgereinigten $F(ab)_2$-Teile, von homologen oder monoklonalen Immunglobulinen der Klassen G1, G2, G3 und/oder G4 und/oder IGM, IGE, IGD und/oder IGA, die selektiv wirksam sind gegen die spezifischen Antigene oder antigenen Determinanten der jeweiligen krankheitserregenden Keime, insbesondere Viren, Bakterien und Pilze, sowie der phathogenen Stoffwechselprodukte, Toxine, Fettkörper und Arzneimittel.

2. Medizinisches Gerät nach Anspruch 1, dadurch gekennzeichnet, daß es sich bei dem Kunststoff handelt um ein Polyolefin, Polyester, Polyäther, Polyamid, Polyimid, Polyurethan, Polyvinylchlorid, Polystyrol, Polysulfon, Polyacrylat, Polymethacrylat, Polypropylen, Polyvinylpyrrolidon, ein Fluorpolymeres, ein Derivat dieser Verbindungen oder um eine Mischung oder ein Copolymeres davon oder einen Silikonkautschuk.

3. Medizinisches Gerät nach Anspruch 1 oder 2, dadurch gekennzeichnet, daß die Kunststoffoberfläche einen Hauftvermittler, insbesondere einen solchen aus der Gruppe Bromcyan, Thiophosgen und Thionylchlorid, aufweist.

4. Medizinisches Gerät nach einem der Ansprüche 1 bis 3, dadurch gekennzeichnet, daß es sich dabei handelt um ein Filter, insbesondere ein Kompaktfilter, Membranfilter, Füllkörperfilter, Molekularsiebfilter oder Kapillarfilter, zur Abtrennung von krankheitserregenden Keimen, insbesondere Viren, Bakterien und Pilzen, sowie pathogenen Stoffwechselprodukten, Toxinen, Fettkörpern und Arzneimitteln aus Blut, dessen Oberfläche besetzt ist mit den $F(ab)_2$-Teilen von homologen oder monoklonalen Immunglobulinen, vorzugsweise solchen, die durch Affinitätschromatographie nachgereinigt worden sind, der Klassen G1, G2, G3 und/oder G4 und/oder IGM, IGE, IGD und/oder IGA, die spezifisch wirksam sind gegen die spezifischen Antigene oder antigenen Determinanten der jeweiligen krankheitserregenden Keime, insbesondere Viren, Bakterien und Pilze,

sowïe der pathogenen Stoffwechselprodukte, Toxine, Fettkörper und Arzneimittel.

## Revendications

1. Appareillage médical, en particulier filtre, canule, cathéter ou implant, en matière plastique ou en métal recouvert de matière plastique ou en verre, à l'aide duquel, à l'intérieur et à l'extérieur du corps humain, peuvent être fixés, et ainsi rendus inoffensifs, des germes pathogènes, en particulier des virus, des bactéries et des champignons, ainsi que des produits de métabilisme pathogènes, des toxines, des corps gras et des anticorps, appareillage pour lequel la surface de matière plastique est réalisée avec des anticorps fixés ou reliés avec elle par covalence, caractérisé en ce qu'il s'agit, en ce qui concerne les anticorps, de fragments de type F(ab)$_2$, de préférence les fragments de type F(ab)$_2$ qui ont été purifiés par chromatographie par affinité, d'immunoglobulines homologues ou monoclonales, des classes G1, G2, G3 et/ou G4 et/ou IgM, IgE, IgD et/ou IgA, qui sont spécifiquement efficaces contre les antigènes spécifiques ou les déterminants antigéniques des différents germes pathogènes, en particulier les virus, bactéries et champignons, ainsi que les produits de métabolisme pathogènes, les toxines, les corps gras et les médicaments.

2. Appareillage médical suivant la revendication 1, caractérisé en ce que, pour la matière plastique, il s'agit d'une polyoléfine, d'un polyester, d'un polyéther, d'un polyamide, d'un polyuréthane, d'un chlorure de polyvinyle, d'un polystyrène, d'un polysulfone, d'un polyacrylate, d'un polyméthacrylate, d'un polypropylène, d'un polyvinylpyrrolidone, d'un polymère fluoré, d'un dérivé de ces composés ou d'un mélange ou d'un copolymère de ceux-ci ou d'un caoutchouc silicone.

3. Appareillage médical suivant la revendication 1 ou la revendication 2, caractérisé en ce que la surface externe en matière plastique présente un agent de fixation, en particulier un agent provenant du groupe cyanobrome, thiophosgène et chlorure de thionyle.

4. Appareillage médical suivant l'une quelconque des revendications 1 à 3, caractérisé en ce qu'il s'agit, dans ce cas, d'un filtre, en particulier d'un filtre compact, d'un filtre à membrane, d'un filtre à corps de remplissage, d'un filtre à tamis moléculaire ou d'un filtre capillaire, pour éliminer les germes pathogènes, en particulier les virus, bactéries et champignons, ainsi que les produits de métabolisme pathogènes, les toxines, les corps gras et les médicaments provenant du sang, filtre dont la surface externe est revêtue de fragments de type F(ab)$_2$ d'immunoglobulines homologues ou monoclonales, de préférence ceux qui ont été purifiés par chromatographie par affinité, des classes G1, G2, G3 et/ou G4 et/ou IgM, IgE, IgD et/ou IgA, qui sont spécifiquement efficaces contre les antigènes spécifiques ou les déterminants antigéniques des différents germes pathogènes, en particulier les virus, bactéries et champignons, ainsi que les produits de métabolisme pathogènes, les toxines, les corps gras et les médicaments.

## Claims

1. Medical device, in particular filter, cannula, catheter or implant, consisting of synthetic material or metal or glass coated with synthetic material for fixing pathogenic germs, in particular viruses, bacteria and fungi, as well as pathogenic metabolic products, toxins, aliphatic compounds and medicines, in which the surface of the synthetic material is provided with antibodies bonded securely thereto or connected thereto in a covalent manner, characterised in that the antibodies are F(ab)$_2$-parts, preferably the F(ab)$_2$-parts repurified by affinity chromatography, of homologous or monoclonal immunoglobulins of classes G1, G2, G3 and/or G4 and/or IGM, IGE, IGD and/or IGA, which are selectively effective against the specific antigens or antigenic determinants of the respective pathogenic germs, in particular viruses, bacteria and fungi, as well as of the pathogenic metabolic products, toxins, aliphatic compounds and medicines.

2. Medical device according to Claim 1, characterised in that the synthetic material is a polyolefin, polyester, polyether, polyamide, polyimide, polyurethane, polyvinyl chloride, polystyrene, polysulphone, polyacrylate, polymethacrylate, polypropylene, polyvinylpyrolidone, a fluoropolymer, a derivative of these compounds or a mixture or a copolymer thereof or a silicone rubber.

3. Medical device according to Claim 1 or 2, characterised in that the surface of the synthetic material comprises an agent promoting adhesion, in particular from the group comprising cyanogen bromide, thiophosgene and thionyl chloride.

4. Medical device according to one of Claims 1 to 3, characterised in that in this case it is a filter, in particular a compact filter, membrane filter, filling body filter, molecular sieve filter or capillary filter, for the separation of pathogenic germs, in particular viruses, bacteria and fungi, as well as pathogenic metabolic products, toxins, aliphatic compounds and medicines from blood, the surface of which filter is provided with F(ab)$_2$-parts of homologous or monoclonal immunoglobulins, preferably those which have been repurified by affinity chromatography, of classes G1, G2, G3 and/or G4 and/or IGM, IGE, IGD and/or IGA, which are specifically effective against the specific antigens or antigenic determinants of the respective pathogenic germs, in particular viruses, bacteria and fungi, as well as of pathogenic metabolic products, toxins, aliphatic compounds and medicines.